# EUROPEAN PATENT SPECIFICATION

(11) **EP 2 175 915 B1**
(45) Date of publication and mention of the grant of the patent: **21.02.2018**
(21) Application number: 08788234.6
(22) Date of filing: 28.07.2008
(51) Int. Cl.: A61M 5/20, A61M 5/32

(54) **INJECTION DEVICE WITH LOCKING MECHANISM FOR SYRINGE CARRIER**
INJEKTIONSVORRICHTUNG MIT SPERRMECHANISMUS FÜR SPRITZENTRÄGER
DISPOSITIF D'INJECTION À MÉCANISME DE VERROUILLAGE POUR PORTE-SERINGUE

(30) Priority: 08.08.2007 GB 0715460
(43) Date of publication of application: 21.04.2010
(73) Proprietor: Cilag GmbH International, 6300 Zug (CH)
(72) Inventor: JENNINGS, Douglas, Ivan, Royston, Hertfordshire SG8 7XU (GB)
(74) Representative: Tunstall, Christopher Stephen
(86) International application number: PCT/GB2008/002578
(87) International publication number: WO 2009/019437

(56) References cited:
- EP-A- 1 586 341
- WO-A-03/013632
- WO-A-2007/036676
- WO-A-2008/075033
- US-A1- 2005 203 466

## Description

### Field of the Invention

The present invention relates to an injection device of the type which has a syringe and which extends the syringe, discharges its contents and then retracts it automatically.

### Background of the Invention

Injection devices are shown in WO 95/35126 and EP-A-0 516 473. These devices employ a drive spring and some form of release mechanism that releases the syringe from the influence of the drive spring once its contents are supposed to have been discharged, to allow it to be retracted by a return spring.

Generally, the return spring is relatively weak, since its restoring force must be overcome by the drive spring, even while the drive spring is doing work on the various components of the injection device and the syringe during an injection cycle. This may give rise to a problem when the injection device is used with sealed hypodermic syringes, which typically have a hermetically sealed cover, needle shield or "boot" that covers the hypodermic needle and maintains the sterility of the syringe contents. Naturally, it is necessary to maintain the sterility of the syringe contents up to the point of administration, which for devices that are designed to be disposable, as many will be, means that the boot must be removed with the syringe inside the injection device.

Typically, the action required to remove the boot from the syringe is simply to pull the boot away from the syringe, which requires a force in excess of 20N. This is significantly greater than the restoring force of the return spring, so the syringe will be pulled out of the injection device as the boot is removed and, when the boot comes away, it will snap back into place. This is not the best way to handle the syringe. The shock could damage it, the needle could be damaged and there may be problems re-engaging the syringe with those components of the injection device designed to act upon it. Even in cases where there is no return spring, for example where the syringe is held in place by friction with components of the injection device, the problem will still arise of relocating the syringe onto those components of the injection device designed to act upon it.

Moreover, there is a problem with having the syringe generally moveable in a direction out of the injection device. Accidental activation of the drive spring by mechanical failure of the drive spring's release mechanism (e.g. a trigger) can occur, for example by dropping the device on a hard surface. This accidental activation could cause the syringe to be extended unintentionally out of the device and its contents to be ejected. This could expose the needle of the syringe and increase the risk of inadvertent ski puncturing and/or infection.

WO 2007/036676 describes an injection device comprising a releasable locking mechanism which retains the syringe in a retracted position, released by a sleeve projecting from the housing. A removable threaded cap closes the housing and covers the exit aperture and sleeve.

WO 03/01362 describes another injection device containing locking fingers and ribs on a syringe carrier which engage with ribs on a shroud which surrounds the syringe and syringe carrier.

### Summary of the Invention

The injection device of the present invention is designed to deal with the aforementioned problems.

In a first aspect of the present invention, there is provided an injection device comprising:
a housing adapted to receive a syringe having a discharge nozzle, the syringe being movable along a longitudinal axis of the housing between a retracted position in which the discharge nozzle is contained within the housing and an extended position in which the discharge nozzle extends from the housing through an exit aperture;
a cap removably located over the exit aperture;
a syringe carrier adapted to support the syringe as it is advanced; and
a needle shield removably located over the discharge nozzle and connected to the cap; and
a locking component which is adapted to prevent, in an engaged position when the cap is located on the housing and during removal of the cap from the housing, movement of the syringe carrier and/or syringe towards the exit aperture relative to the housing,
wherein the needle shield becomes removed from the discharge nozzle during removal of the cap from the housing.

In this way, movement of the syringe carrier and syringe in a forward direction (i.e. towards the exit aperture) is inhibited until the cap has been removed, thereby preventing damage to the syringe and/or its contents. Since the locking component operates in conjunction with the cap, there is an automatic release of the locking mechanism when the cap is removed so that the injection device can be operated following removal of the cap.

Preferably, the cap comprises a shield retainer which grips the needle shield, so that the needle shield becomes removed from the discharge nozzle during removal of the cap from the housing.

Advantageously, the locking component is adapted to prevent movement of the syringe carrier towards the exit aperture during removal of the cap from housing only when the needle shield is not released from the discharge nozzle.

The cap comprises a body and a sleeve located within the body and fixed relative to the body.

The locking component is maintained in its engaged position by the sleeve of the cap during removal of the cap from the housing along the longitudinal axis.

The locking component comprises at least one beam having a first abutment surface engageable with a second abutment surface located on the syringe carrier when the locking component is in its engaged position.

In one embodiment of the present invention, the at least one beam is connected to the housing at an end of the beam opposite the first abutment surface and on the housing at a point located away from the second abutment surface towards the exit aperture.

Preferably, the beam comprises a slot in which the sleeve of the cap resides when the locking component is in its engaged position and in which the sleeve is not so engaged when the cap is removed.

Preferably, the shield retainer is located within the sleeve.

In a second aspect of the present invention, there is provided a method of removing a cap from an injection device having a housing and a syringe located in the housing, the syringe moveable along a longitudinal axis of the housing between a retracted position in which the discharge nozzle is contained within the housing and an extended position in which the discharge nozzle extends from the housing through an exit aperture, the cap being connected to a needle shield of the syringe, the method comprising:
moving the cap linearly along the longitudinal axis such that the needle shield is moved along the longitudinal axis out of the exit aperture, thereby releasing it from the discharge nozzle, whilst a locking component in the cap prevents movement of the syringe along the longitudinal axis,
wherein when the needle shield has been released from the discharge nozzle, further movement of the cap along the longitudinal axis to expose the exit aperture releases the locking component so that the discharge nozzle can be moved towards its extended position during operation of the injection device.

### Brief Description of the Drawings

The invention will now be described by way of example with reference to the accompanying drawings, in which:
Fig. 1a is a right-side view of the injection device according to the present invention;
Fig. 1b is a perspective view of the injection device of Fig. 1 with its cap removed;
Fig. 1c is a perspective view of the cap of the injection device of Fig. 1;
Fig. 2a is an exploded right-side view of the injection device of Fig. 1;
Fig. 2b is a right-side view of the assembled components of the injection device of Fig. 1;
Fig. 2c is a perspective view of a multi-component drive used in the injection device of Fig. 1
Fig. 3 is a cross-sectional view of the injection device of Fig. 1.

### Detailed Description of the Drawings

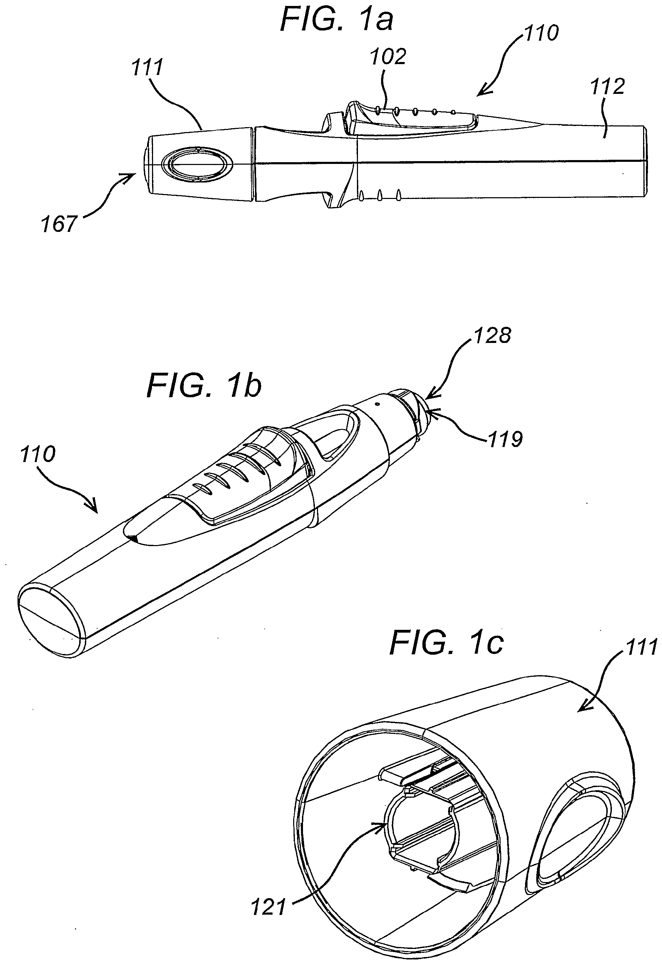

Fig. 1 a is a right-side view of an injection device 110 according to the present invention. The injection device 110 has a housing 112, a cap 111 which is removable from a proximal end 167 of the housing 112 and a trigger button 102. Other parts of the device will be described in greater detail below.

Fig. 1b is a perspective view of the injection device 110 according to the present invention with the cap (not shown) removed from its end. The end of the housing 112 has an exit aperture 128, from which the end of a sleeve 119 can be seen to emerge.

Fig. 1c is a perspective view of the cap 111 of the injection device 110 according to the present invention. The cap 111 has a central boss 121 that fits within the sleeve 119 when the cap 111 is installed on the housing 112.

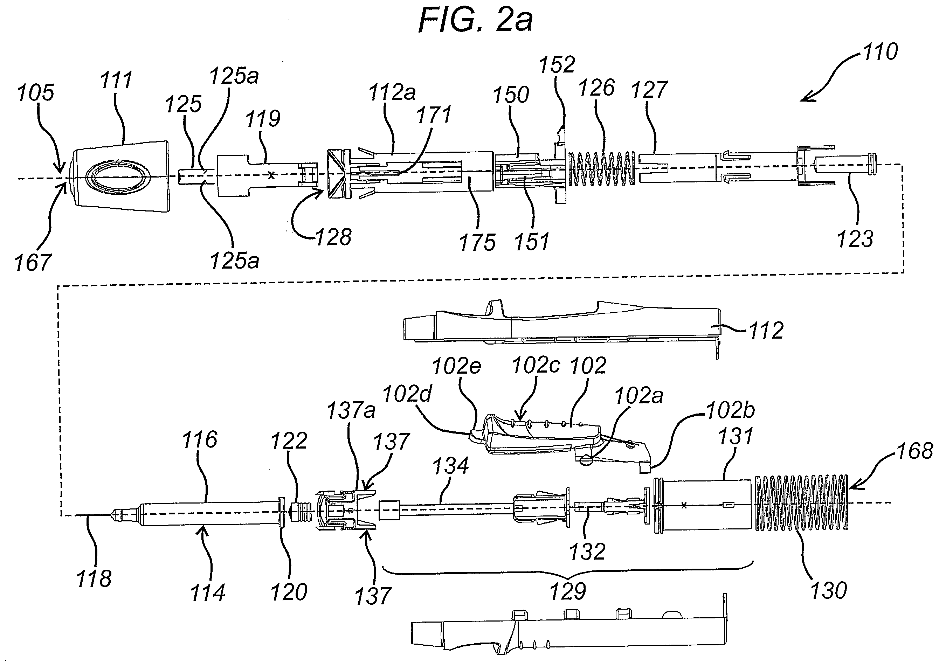

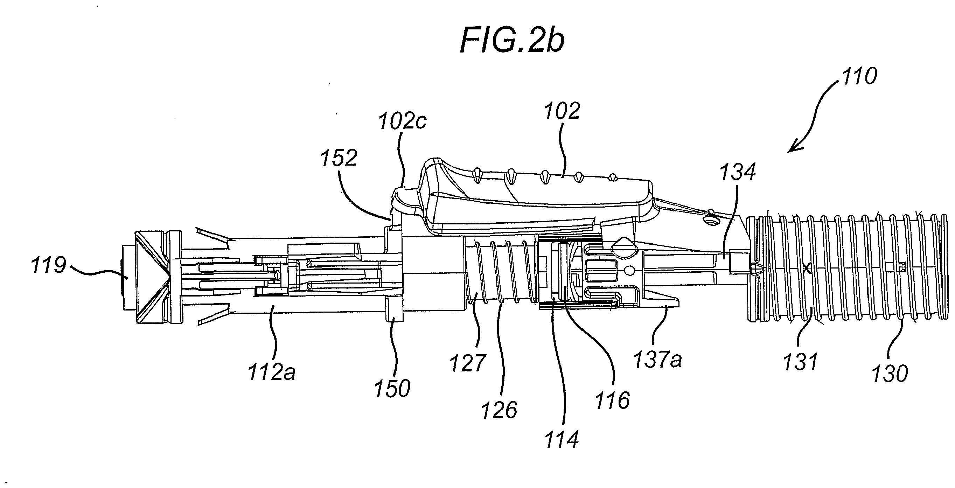

Fig. 2a is an exploded right-side view of the components of the injection device 110 according to the present invention and Fig. 2b is a right-side view of the assembled components of the injection device 110 according to the present invention without the housing 112 or cap 111.

As illustrated, the injection device 110 comprises a hypodermic syringe 114 of conventional type, including a syringe body 116 terminating at one end in a discharge nozzle, specifically a hypodermic needle 118, and at the other in a flange 120. The conventional plunger that would normally be used to discharge the contents of the syringe 114 manually has been removed and replaced with a drive element (referred to below as the second drive element 134) that contacts a bung 122 in the syringe 114. The bung 122 constrains a drug (not shown) to be administered within the syringe body 116. Whilst the syringe illustrated is of hypodermic type, this need not necessarily be so. Transcutaneous or ballistic dermal and subcutaneous syringes may also be used with the injection device of the present invention.

As illustrated, the injection device 110 includes a return spring 126 that biases the syringe 114 from an extended position in which the needle 118 extends from the aperture 128 in a case nose 112a of the housing 112 to a retracted position in which the needle 118 is contained within the housing 112. The return spring 126 acts on the syringe 114 via a syringe carrier 127. The syringe 114 is moveable along a longitudinal axis 105 of the injection device 110 which extends centrally along the length of the injection device 110 from the exit aperture 128 at its proximal end 167 to a distal end 168.

Contained within the housing at its distal end 168 is an actuator, which here takes the form of a compression drive spring 130. Drive from the drive spring 130 is transmitted via a multi-component drive 129 to the syringe 114 to advance it from its retracted position to its extended position and discharge its contents through the needle 118. The drive 129 accomplishes this task by acting directly on the drug and the syringe 114. Hydrostatic forces acting through the drug and, to a lesser extent, static friction between the bung 122 and the syringe body 116 initially ensure that they advance together, until the return spring 126 bottoms out on the syringe carrier 127 or meets some other obstruction (not shown) that retards its motion.

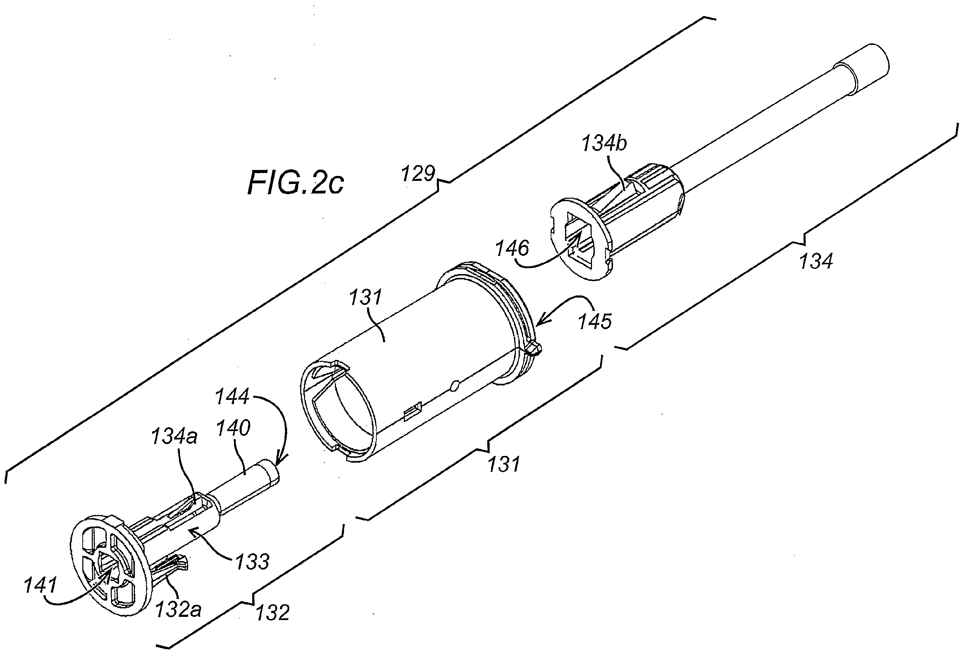

Fig. 2c is an exploded perspective view of the multi-component drive 129. The multi-component drive 129 between the drive spring 130 and the syringe 114 consists of three principal components. A drive sleeve 131 takes drive from the drive spring 130 and transmits it to a delay piston 133 on a first drive element 132. This in turn transmits drive to the second drive element 134.

As will be seen from Fig. 2c, the first drive element 132 includes a hollow stem 140, the inner cavity of which forms a collection chamber 141 in communication with a vent 144 that extends from the collection chamber 141 through the end of the stem 140. The second drive element 134 includes a blind bore 146 that is open at one end to receive the stem 140 and closed at the other. As will be appreciated, the bore 146 and the stem 140 define a fluid reservoir within which a damping fluid is contained.

The trigger button 102 is provided on the side of the housing 112 which, when in an engaged position with a proximal end 145 of the drive sleeve 131, serves to retain the drive spring 130 in a compressed state by contact between locking surface 102b and the drive sleeve 131 when the trigger button 102 is in an unactuated position. The trigger button 102 can pivot on the housing 112 via pivot 102a. When downwards pressure is applied to the trigger button 102 at an activation surface 102c (i.e. pressure directed into the housing 112), the locking surface 102b moves upwards in a direction away from the longitudinal axis 105. In this actuated position of the trigger button 102, the locking surface 102b is decoupled from the drive sleeve 131, thereby allowing the drive sleeve 131 to move relative to the housing 112 towards the exit aperture 128 under the influence of the drive spring 130.

The sliding sleeve 119 is moveable from its extended position (as shown in Fig. 1b) where it protrudes out of the exit aperture 128 into a retracted position in the case nose 112a of the housing 112. The sliding sleeve 119 is connected to a trigger button lock element 150 which has resilient arms 151 which bias the sliding sleeve 119 into its extended position in which its end protrudes from the end of the case nose 112a. Thus, application of pressure to the end of the sliding sleeve 119, for example by pressing the end of the sliding sleeve 119 against tissue, causes it to move into its retracted position into the housing 112; release of the pressure causes the sliding sleeve 119 to move into its extended position under bias from the resilient arms 151 acting against a side wall of the housing 112. The trigger button lock element 150 has a trigger button lock protrusion 152 which contacts with the end of a trigger button protrusion 102d on the trigger button 102 when the sliding sleeve is in its extended position. The trigger button protrusion 102 extends in a direction which is generally parallel to the longitudinal axis 105 of the injection device 110. The trigger button lock protrusion 152 extends in a direction which is generally perpendicular to the longitudinal axis 105 towards the trigger button protrusion 102d. The trigger button protrusion 102d has an aperture 102e which can move over the top of the trigger button lock protrusion 152 when the trigger button lock element 150 has been moved away from the exit aperture 128 (i.e. when the sliding sleeve 119 has been moved into the exit aperture 128 into its retracted position). In this position, the trigger button 102 can be moved into its deactivated position by rotating the trigger button 102 about the pivot 102a in the direction of the pressure applied to the pressure surface 102c. Thus, the trigger button lock element 150 and the sliding sleeve 119 act together to lock the trigger button 102 in its activated position (i.e. the locking surface 102b contacts the end of the drive sleeve 131 preventing it from moving towards the exit aperture 128 under the bias of the compressed drive spring 130).

When the sliding sleeve 119 has been moved into a position in which it is retracted into the housing 112 (i.e. into its unlocked position) and the trigger button 102 has been rotated into its deactivated position, the operation of the device 110 is then as follows.

Initially, the drive spring 130 moves the drive sleeve 131, the drive sleeve 131 moves the first drive element 132 and the first drive element 132 moves the second drive element 134, in each case by acting through flexible latch arms 132a, 134a, 134b. The second drive element 134 moves and, by virtue of static friction and hydrostatic forces acting through the drug (not shown), moves the syringe body 116 and syringe carrier 127 against the action of the return spring 126. The return spring 126 compresses and the hypodermic needle 118 emerges from the exit aperture 128 of the housing 112. This continues until the return spring 126 bottoms out or the syringe body 116 meets some other obstruction (not shown) that retards its motion. Because the static friction between the second drive element 134 and the syringe body 116 and the hydrostatic forces acting through the drug (not shown) to be administered are not sufficient to resist the full drive force developed by the drive spring 130, at this point the second drive element 134 begins to move within the syringe body 116 and the drug (not shown) begins to be discharged. Dynamic friction between the second drive element 134 and the syringe body 116 and hydrostatic forces acting through the drug (not shown) to be administered are, however, sufficient to retain the return spring 126 in its compressed state, so the hypodermic needle 118 remains extended.

Before the second drive element 134 reaches the end of its travel within the syringe body 116, so before the contents of the syringe have fully discharged, the flexible latch arms 134a, 134b linking the first and second drive elements 132, 134 reach a constriction 137 provided on a latch actuator element 137a which is fixed to the end of the syringe carrier 127. The constriction 137 moves the flexible latch arms 134a, 134b inwards from the position shown in Fig. 2c to a position at which the flexible latch arms 134a, 134b no longer couple the first drive element 132 to the second drive element 134, aided by the bevelled surfaces on the constriction 137. Once this happens, the first drive element 132 acts no longer on the second drive element 134, allowing the first drive element 132 to move relative to the second drive element 134.

Because the damping fluid is contained within a reservoir (not shown) defined between the end of the first drive element 132 and the blind bore 146 in the second drive element 134, the volume of the reservoir will tend to decrease as the first drive element 132 moves relative to the second drive element 134 when the former is acted upon by the drive spring 130. As the reservoir collapses, damping fluid is forced through the vent 144 into the collection chamber 141. Thus, once the flexible latch arms 134a, 134b have been released, the force exerted by the drive spring 130 does work on the damping fluid, causing it to flow though the constriction formed by the vent 144, and also acts hydrostatically through the fluid and through friction between the first and second drive elements 132, 134, thence via the second drive element 134. Losses associated with the flow of the damping fluid do not attenuate the force acting on the body of the syringe to a great extent. Thus, the return spring 126 remains compressed and the hypodermic needle remains extended.

After a time, the second drive element 134 completes its travel within the syringe body 116 and can go no further. At this point, the contents of the syringe 114 are completely discharged and the force exerted by the drive spring 130 acts to retain the second drive element 134 in its terminal position and to continue to cause the damping fluid to flow though the vent 144, allowing the first drive element 132 to continue its movement.

Before the reservoir of fluid is exhausted, the flexible latch arms 132a linking the drive sleeve 131 with the first drive element 132 reach another constriction (not shown) within the housing 112. This constriction moves the flexible latch arms 132a inwards from the position shown to a position at which they no longer couple the drive sleeve 131 to the first drive element 132, aided by bevelled surfaces on the constriction. Once this happens, the drive sleeve 131 acts no longer on the first drive element 132, allowing them to move relative each other. At this point, of course, the syringe 114 is released, because the forces developed by the drive spring 130 are no longer being transmitted to the syringe 114, and the only force acting on the syringe will be the return force from the return spring 126. Thus, the syringe 114 is now returned to its retracted position and the injection cycle is complete.

All this takes place, of course, only once the cap 111 has been removed from the end of the housing 112. The end of the syringe is sealed with a boot 123. The central boss 121 of the cap 111 that fits within the sleeve 119 when the cap 111 is installed on the housing 112 comprises a retainer element 125 which is fixed into the boss 121. The retainer element 125 comprises resilient protrusions 125a which are directed away from the exit aperture 128. These resilient protrusions 125a deform as the cap 111 is inserted onto the housing 112 over a needle shield or rubber boot 123. The protrusions 125a then grip the boot 123 tightly so that the ends of the protrusions are slightly embedded in the boot 123 which might be made from rubber. This means that, as the cap 111 is pulled off the housing 112, the boot 123 is pulled away from the syringe 114 with the cap 111.

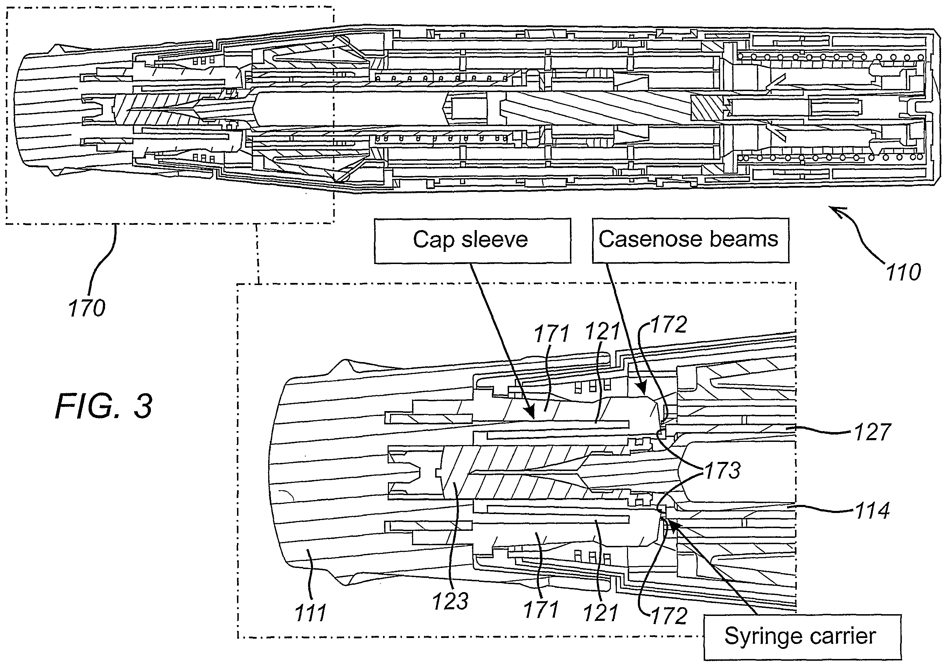

Fig. 2a also shows a locking mechanism 170 formed integrally with the case nose 112a and Fig. 3 shows how the locking mechanism 170 is integrated with the injection device 110 of the present invention.

The locking mechanism 170 is located on the case nose 112a. The locking mechanism is formed by two beams 171, each forming a separate locking component. Each beam 171 is adapted to prevent movement of the syringe carrier 127 towards the exit aperture 128 during removal of the cap 111 from housing 112. This is achieved by each beam 171 having a first abutment surface 172 which is engageable with a second abutment surface 173 located on a proximal end of the syringe carrier 127 when the locking component is in its engaged position. Each beam 171 is resiliently biased so that when the cap 111 is not connected to the proximal end 167 of the housing 112, the first and second abutment surfaces 172, 173 are not in contact with each other. In this arrangement, each beam 171 has moved outwardly, i.e. away from the longitudinal axis 105 towards the housing 112 so that the abutment surfaces 172, 173 are no longer in contact with each other.

The central boss 121 of the cap 111 is in the form of a sleeve which is engageable with a slot 174 in each beam 171 to hold the beam in its engaged position so that the first and second abutment surfaces 172, 173 are in contact with each other, thereby preventing movement of the syringe carrier 127 in a forward direction towards the exit aperture 128. When the cap 111 is removed, the boss 121 slides out of the slots on the beams so that the beams are no longer held in their engaged positions with respect to the syringe carrier 127. This means that each beam 171 can move outwardly with respect to the longitudinal axis 105 under force generated by the resilience in the beams themselves so that the abutment surfaces 172, 173 are no longer in contact with each other.

As shown in Fig. 3, each beam 171 is connected to the housing 112 at a proximal end of the beam 171 opposite the first abutment surface 172 and on the housing 112 at a point located away from the second abutment surface 173 towards the exit aperture 128.

## Claims

1. An injection (110) device comprising:
a syringe (114) having a discharge nozzle (118);
a housing (112) adapted to receive the syringe the syringe being movable along a longitudinal axis of the housing (112) between a retracted position in which the discharge nozzle is contained within the housing and an extended position in which the discharge nozzle extends from the housing through an exit aperture;
a cap (111) removably located over the exit aperture (128), wherein the cap (111) comprises a body and a sleeve (121) located within the body and fixed relative to the body;
a syringe carrier (127) adapted to support the syringe as it is advanced; and
a needle shield (123) removably located over the discharge nozzle and connected to the cap, wherein the needle shield becomes removed from the discharge nozzle during removal of the cap from the housing; and
a locking component (171) which is adapted to prevent, in an engaged position when the cap is located on the housing and during removal of the cap from the housing, movement of the syringe carrier and/or syringe towards the exit aperture relative to the housing,
**characterised in that** the locking component comprises at least one beam (171) having a first abutment surface engageable (172) with a second abutment surface (173) located on the syringe carrier when the locking component is in its engaged position,
wherein the locking component is maintained in its engaged position by the sleeve (121) of the cap during removal of the cap from the housing along the longitudinal axis.

2. The injection device of claim 1, wherein the cap further comprises a shield retainer (125) adapted to grip the needle shield, such that the needle shield becomes removed from the discharge nozzle during removal of the cap from the housing.

3. An injection device according to claim 1 or claim 2, wherein the locking component (171) is adapted to prevent movement of the syringe carrier towards the exit aperture during removal of the cap (111) from housing (112) only when the needle shield (123) is not released from the discharge nozzle (118).

4. An injection device according to any of the preceding claims, wherein the at least one beam (171) is connected to the housing at an end of the beam opposite the first abutment surface (172) and on the housing at a point located away from the second abutment surface (173) towards the exit aperture.

5. An injection device according to any of the preceding claims, wherein the beam comprises a slot in which the sleeve (121) of the cap resides when the locking component is in its engaged position.

6. An injection device according to claim 4 when dependant on claim 2, wherein the shield retainer (125) is located within the sleeve.

7. A method of removing a cap (111) from an injection device (110) having a housing (112) and a syringe (114) located in the housing, the syringe moveable along a longitudinal axis of the housing between a retracted position in which the discharge nozzle (118) is contained within the housing and an extended position in which the discharge nozzle extends from the housing through an exit aperture (128), the cap being connected to a needle shield (123) of the syringe, the method comprising:
moving the cap linearly along the longitudinal axis such that the needle shield is moved along the longitudinal axis out of the exit aperture, thereby releasing it from the discharge nozzle, whilst a locking component in the cap prevents movement of the syringe along the longitudinal axis,
**characterised in that** when the needle shield has been released from the discharge nozzle, further movement of the cap along the longitudinal axis to expose the exit aperture releases the locking component so that the discharge nozzle can be moved towards its extended position during operation of the injection device.

## Patentansprüche

1. Injektionsvorrichtung (110), umfassend:
eine Spritze (114) mit einer Austrittsdüse (118);
ein Gehäuse (112), das zur Aufnahme der Spritze ausgelegt ist, wobei die Spritze entlang einer Längsachse des Gehäuses (112) zwischen einer zurückgezogenen Position, in der die Austrittsdüse im Gehäuse enthalten ist, und einer ausgezogenen Position, in der sich die Austrittsdüse vom Gehäuse durch eine Austrittsöffnung erstreckt, beweglich ist;
eine Kappe (111), die entfernbar über der Austrittsöffnung (128) angeordnet ist, wobei die Kappe (111) einen Körper und eine Hülse (121) umfasst, die im Körper angeordnet ist und bezüglich des Körpers fixiert ist;
einen Spritzenträger (127), der zur Unterstützung der Spritze ausgelegt ist, wenn diese vorgeschoben wird; und
ein Nadelschild (123), das entfernbar über der Austrittsdüse angeordnet ist und mit der Kappe verbunden ist, wobei das Nadelschild während der Entfernung der Kappe vom Gehäuse von der Austrittsdüse entfernt wird; und
eine Sperrkomponente (171), die in einer Eingriffsposition, wenn die Kappe auf dem Gehäuse angeordnet ist, und während der Entfernung der Kappe vom Gehäuse zur Verhinderung einer Bewegung des Spritzenträgers und/oder der Spritze zur Austrittsöffnung bezüglich des Gehäuses ausgelegt ist,
**dadurch gekennzeichnet, dass** die Sperrkomponente mindestens einen Stab (171) mit einer ersten Anlagefläche (172), die mit einer zweiten Anlagefläche (173) auf dem Spritzenträger in Eingriff gebracht werden kann, wenn sich die Sperrkomponente in ihrer Eingriffsposition befindet, umfasst,
wobei die Sperrkomponente von der Hülse (121) der Kappe während der Entfernung der Kappe vom Gehäuse entlang der Längsachse in ihrer Eingriffsposition gehalten wird.

2. Injektionsvorrichtung nach Anspruch 1, wobei die Kappe ferner einen Schildhalter (125) umfasst, der zum Greifen des Nadelschilds ausgelegt ist, so dass das Nadelschild während der Entfernung der Kappe vom Gehäuse von der Austrittsdüse entfernt wird.

3. Injektionsvorrichtung nach Anspruch 1 oder Anspruch 2, wobei die Sperrkomponente (171) dazu ausgelegt ist, eine Bewegung des Spritzenträgers zur Austrittsöffnung während der Entfernung der Kappe (111) vom Gehäuse (112) nur dann zu verhindern, wenn das Nadelschild (123) nicht von der Austrittsdüse (118) freigegeben ist.

4. Injektionsvorrichtung nach einem der vorhergehenden Ansprüche, wobei der mindestens eine Stab (171) mit dem Gehäuse an einem Ende des Stabs gegenüber der ersten Anlagefläche (172) und auf dem Gehäuse an einem Punkt, der entfernt von der zweiten Anlagefläche (173) zur Austrittsöffnung angeordnet ist, verbunden ist.

5. Injektionsvorrichtung nach einem der vorhergehenden Ansprüche, wobei der Stab einen Schlitz umfasst, in dem die Hülse (121) der Kappe sitzt, wenn sich die Sperrkomponente in ihrer Eingriffsposition befindet.

6. Injektionsvorrichtung nach Anspruch 4 in Abhängigkeit von Anspruch 2, wobei der Schildhalter (125) in der Hülse angeordnet ist.

7. Verfahren zur Entfernung einer Kappe (111) von einer Injektionsvorrichtung (110) mit einem Gehäuse (112) und einer im Gehäuse angeordneten Spritze (114), wobei die Spritze entlang einer Längsachse des Gehäuses zwischen einer zurückgezogenen Position, in der die Austrittsdüse (118) im Gehäuse enthalten ist, und einer ausgezogenen Position, in der sich die Austrittsdüse vom Gehäuse durch eine Austrittsöffnung (128) erstreckt, beweglich ist, wobei die Kappe mit einem Nadelschild (123) der Spritze verbindbar ist, wobei das Verfahren das Folgende umfasst:
lineares Bewegen der Kappe entlang der Längsachse, so dass das Nadelschild entlang der Längsachse aus der Austrittsöffnung bewegt wird, wodurch es von der Austrittsdüse freigegeben wird, während eine Sperrkomponente in der Kappe eine Bewegung der Spritze entlang der Längsachse verhindert,
**dadurch gekennzeichnet, dass**, wenn das Nadelschild von der Austrittsöffnung freigegeben wurde, eine weitere Bewegung der Kappe entlang der Längsachse zum Freilegen der Austrittsöffnung die Sperrkomponente freigibt, so dass die Austrittsdüse während der Bedienung der Injektionsvorrichtung in ihre ausgezogene Position bewegt werden kann.

## Revendications

1. Dispositif d'injection (110), comprenant:
une seringue (114) présentant une buse de décharge (118) ;
un boîtier (112) adapté pour recevoir la seringue, la seringue étant déplaçable le long d'un axe longitudinal du boîtier (112) entre une position rétractée, dans laquelle la buse de décharge est contenue à l'intérieur du boîtier, et une position étendue, dans laquelle la buse de décharge s'étend à partir du boîtier à travers une ouverture de sortie;
une coiffe (111) disposée de façon amovible sur l'ouverture de sortie (128), dans lequel la coiffe (111) comprend un corps et un manchon (121) situé à l'intérieur du corps et fixé par rapport au corps;
un porte-seringue (127) adapté pour supporter la seringue lorsqu'elle est avancée; et
un écran d'aiguille (123) disposé de façon amovible sur la buse de décharge et connecté à la coiffe, dans lequel l'écran d'aiguille est enlevé de la buse de décharge pendant l'enlèvement de la coiffe du boîtier; et
un composant de verrouillage (171) qui est adapté pour empêcher, dans une position engagée lorsque la coiffe est disposée sur le boîtier et pendant l'enlèvement de la coiffe du boîtier, le déplacement du porte-seringue et/ou de la seringue en direction de l'ouverture de sortie par rapport au boîtier,
**caractérisé en ce que** le composant de verrouillage comprend au moins une poutre (171) qui présente une première surface de butée (172) engageable avec une seconde surface de butée (173) qui est située sur le porte-seringue lorsque le composant de verrouillage se trouve dans sa position engagée,
dans lequel le composant de verrouillage est maintenu dans sa position engagée par le manchon (121) de la coiffe pendant l'enlèvement de la coiffe du boîtier le long de l'axe longitudinal.

2. Dispositif d'injection selon la revendication 1, dans lequel la coiffe comprend en outre un élément de retenue d'écran (125) adapté pour accrocher l'écran d'aiguille, de telle sorte que l'écran d'aiguille soit enlevé de la buse de décharge pendant l'enlèvement de la coiffe du boîtier.

3. Dispositif d'injection selon la revendication 1 ou la revendication 2, dans lequel le composant de verrouillage (171) est adapté pour empêcher le déplacement du porte-seringue en direction de l'ouverture de sortie pendant l'enlèvement de la coiffe (111) du boîtier (112) uniquement lorsque l'écran d'aiguille (123) n'est pas détaché de la buse de décharge (118).

4. Dispositif d'injection selon l'une quelconque des revendications précédentes, dans lequel ladite au moins une poutre (171) est connectée au boîtier à une extrémité de la poutre opposée à la première surface de butée (172) et sur le boîtier en un point situé à l'écart de la seconde surface de butée (173) en direction de l'ouverture de sortie.

5. Dispositif d'injection selon l'une quelconque des revendications précédentes, dans lequel la poutre comporte une fente dans laquelle le manchon (121) de la coiffe réside lorsque le composant de verrouillage se trouve dans sa position engagée.

6. Dispositif d'injection selon la revendication 4 lorsqu'elle dépend de la revendication 2, dans lequel l'élément de retenue d'écran (125) est situé à l'intérieur du manchon.

7. Procédé pour enlever une coiffe (111) d'un dispositif d'injection (110) comprenant un boîtier (112) et une seringue (114) logée dans le boîtier, la seringue étant déplaçable le long d'un axe longitudinal du boîtier entre une position rétractée, dans laquelle la buse de décharge (118) est contenue à l'intérieur du boîtier, et une position étendue, dans laquelle la buse de décharge s'étend à partir du boîtier à travers une ouverture de sortie (128), la coiffe étant connectée à un écran d'aiguille (123) de la seringue, le procédé comprenant les étapes suivantes:
déplacer la coiffe de façon linéaire le long de l'axe longitudinal de telle sorte que l'écran d'aiguille soit déplacé le long de l'axe longitudinal hors de l'ouverture de sortie, détachant de ce fait celui-ci de la buse de décharge, pendant qu'un composant de verrouillage dans la coiffe empêche tout déplacement de la seringue le long de l'axe longitudinal,
**caractérisé en ce que**, lorsque l'écran d'aiguille a été détaché de la buse de décharge, un déplacement supplémentaire de la coiffe le long de l'axe longitudinal pour exposer l'ouverture de sortie libère le composant de verrouillage de telle sorte que la buse de décharge puisse être déplacée en direction de sa position étendue pendant le fonctionnement du dispositif d'injection.
